# EUROPÄISCHE PATENTSCHRIFT

(11) **EP 0 481 316 B1**
(45) Veröffentlichungstag und Bekanntmachung des Hinweises auf die Patenterteilung: **01.06.1994**
(21) Anmeldenummer: 91117028.0
(22) Anmeldetag: 07.10.1991
(51) Int. Cl.: C07C 209/36

(54) **Verfahren zur Herstellung von 2,2-Bis-(aminophenyl)-propan**
Process for the preparation of 2,2,-bis(aminophenyl)-propane
Procédé pour la préparation du 2,2-bis(aminophényl)-propane

(30) Priorität: 18.10.1990 DE 4033099
(43) Veröffentlichungstag der Anmeldung: 22.04.1992
(73) Patentinhaber: BAYER AG, 51368 Leverkusen (DE)
(72) Erfinder: Buysch, Hans-Josef, Dr., W-4150 Krefeld (DE); Arlt, Dieter, Prof. Dr., W-5000 Köln 80 (DE)

(56) Entgegenhaltungen:
- US-A- 5 037 994
- JOURNAL OF THE AMERICAN CHEMICAL SOCIETY. Bd. 32, März 1930, WASHINGTON, DC US Seiten 1122 - 1127; S.C. HUSSEY ET.AL.: 'A NEW CYCLIC AZOXY COMPOUND'

## Beschreibung

Die Vorliegende Erfindung betrifft ein Verfahren zur Herstellung von 2,2-Bis-(aminophenyl)-propan mit einem Anteil des 4,4′-Isomeren von mindestens 70 % durch Nitrierung von 2,2-Diphenyl-propan und nachfolgende Reduktion.

2,2-Bis-(4-aminophenyl)-propan ist ein interessantes Ausgangsprodukt zur Herstellung von Polymeren, wie Fasern (Makromol. Chem. 32, (1959), 1-12), Epoxidharzen (US 2.989.498; CS 177 664, zitiert nach C.A. 92 (1980), 59 771e), Polyamidimiden (JP 73/11 826, zitiert nach C.A. 80 (1974) 84 401u), oder von Korrosionsschutzmitteln (Vopr. Khim. Khim. Tekhnol. 67 (1982), 65-68, zitiert nach C.A. 100 (1984), 37 873w).

Daher hat man nach geeigneten Synthesen für Diaminodiphenylpropan gesucht. Die bisher beste bekannte Synthese scheint in der Kondensation von Anilin mit Aceton zu bestehen. So erhält man nach US 3.670.024 Diaminodiphenylpropan durch Kondensation von Anilinhydrochlorid mit Aceton. Obwohl die Ausbeute bis zu 73 % der theoretischen Ausbeute betragen kann, hat das Verfahren den Nachteil, daß große Mengen an Natriumchlorid als Beiprodukt anfallen.

Es wurde nun gefunden, daß man sehr hohe Ausbeuten an 2,2-Bis-(aminophenyl)-propan erhält, wenn man 2,2-Diphenylpropan zu 2,2-Bis-(nitrophenyl)-propan nitriert und in einem zweiten Schritt zu 2,2-Bis-(aminophenyl)-propan reduziert. Dabei kann in Ausbeuten von bis über 80 %, jedoch von mindestens 70 % der theoretischen Ausbeute das besonders begehrte 2,2-Bis-(4-aminophenyl)propan erhalten werden. Dies ist in hohem Maße überraschend, denn die Nitrierung und Reduktion analoger Diphenylalkane ergibt wesentlich andere Ausbeuten und Isomerenverteilungen. So erhält man durch Nitrierung von Diphenylmethan nach einem besonders optimierten Verfahren (EP 125 169) 32-55 % (gemäß Ausführungsbeispiel 48 %) des 4,4′-Dinitrodiphenylmethans, wobei eine ganze Reihe von in 2,2′-, 3,3′-Position nitrierten Isomeren mitgebildet werden. Diphenylethan liefert bei der Nitrierung ebenfalls Gemische der verschiedenen Isomeren und nur 33 bis 55 % des 4,4′-Isomeren (J. Am. Chem. Soc. 52 (1930) 1122; EP 149 388).

Die Erfindung betrifft ein Verfahren zur Herstellung von 2,2-Bis-(aminophenyl)-propan mit einem Anteil des 4,4′-Isomeren von mindestens 70 %, das dadurch gekennzeichnet ist, daß man 2,2-Diphenylpropan in einem Lösungsmittel aus der Gruppe der aliphatischen Kohlenwasserstoffe und der aliphatischen Halogenkohlenwasserstoffe mit einem Nitriermittel aus der Rehie 50-100%ige HNO₃, HNO₃/H₂SO₄-Gemische (worin die HNO₃ bis zu 60 Gew.-% H₂O enthalten und die H₂SO₄ bis zu 20 Gew.-% H₂O oder bis zu 20 Gew.-% SO₃ enthalten können) oder Gemische aus H₂SO₄ und Nitraten der (Erd)alkalimetalle, wobei das molare Verhältnis von 2,2-Diphenylpropan zu dem die Nitrogruppe enthaltenden Nitrierreagens 1: (2-15) beträgt, zu 2,2-Bis-(nitrophenyl)-propan nitriert und dieses in einem nachfolgenden Schritt zu 2,2-Bis-(aminophenyl)-propan reduziert.

2,2-Diphenylpropan ist auf verschiedenen Wegen zuganglich. Gute Ausbeuten erhält man beispielsweise durch Umsetzung von α-Chlor-isopropylbenzol mit Benzol in Gegenwart von Lewissauren, insbesondere in Gegenwart von Aluminiumchlorid.

Aufgrund der guten Zuganglichkeit des Ausgangsproduktes Diphenylpropan und der guten Ausbeute an Diamino-di-phenylpropan ist das erfindungsgemäße Verfahren den bisherigen überlegen.

Geeignete Nitrierungsmittel sind beispielsweise konzentrierte und hochkonzentrierte Salpetersauren mit einem Gehalt von 50-100 % HNO₃, beispielsweise die azeotrop siedende wäßrige HNO₃, Gemische aus Salpetersäuren mit Schwefelräuren, wobei die Schwefelsäuren sowohl bis zu 20 Gew.-% Wasser als auch bis zu 20 Gew.-% SO₃ enthalten können, oder Gemische aus Schwefelsäure und Nitraten der (Erd)alkalimetalle, wie Natrium-, Kalium- oder Calciumnitrat. In bevorzugter Weise werden HNO₃/H₂SO₄-Gemische der verschiedenen Konzentrationen (sogenannte Nitriersäure) eingesetzt. In solchen Nitriersäuren kann die HNO₃ bis zu 60 Gew.-% Wasser enthalten und die H₂SO₄ die oben angegebene Zusammensetzung haben.

Diphenylpropan kann sowohl ohne als auch mit Lösungsmittel zur Nitrierung eingesetzt werden. In bevorzugter Weise wird es mit einem Lösungsmittel eingesetzt. Hierfür geeignete Lösungsmittel müssen unter den Reaktionsbedingungen inert und durch Destillation leicht abzutrennen sein. Als Lösungsmittel seien solche aus der Gruppe der aliphatischen Kohlenwasserstoffe, der aliphatischen Halogenkohlenwasserstoffe oder der Nitroaromaten genannt. Einzelne Vertreter sind beispielsweise Cyclohexan, Heptan, Isooctan, Isononan, Isododecan, Dichlormethan, Trichlormethan, Tetrachlormethan, Dichlorpropan, Chlorbutan, Dichlorbutan, Chlorcyclohexan, Nitrobenzol und Nitrotoluole. In bevorzugter Weise werden aliphatische Kohlenwasserstoffe und aliphatische Chlorkohlenwasserstoffe eingesetzt.

Das molare Verhältnis von 2,2-Diphenylpropan zu dem die Nitrogruppe enthaltenden Nitrierreagens beträgt 1:2-15, bevorzugt 1:2-5. Mit einem solchen molaren Verhältnis können die zwei erforderlichen Nitrogruppen in das 2,2-Diphenylpropan eingeführt und damit eine vollständige Umwandlung des Ausgangsproduktes erreicht werden. Grundsätzlich ist es jedoch möglich, auch unterstöchiometrische Mengen an Nitrierreagenz einzusetzen, wenn eine vollständige Umwandlung des Ausgangsproduktes nicht angestrebt wird.

Die Temperatur für die Nitrierung kann in weiten Grenzen, beispielsweise von 0 bis 150°C, schwanken. Innerhalb dieses Temperaturbereiches wird man bei höherkonzentriertem Nitrierreagenz die niedrigeren Bereiche auswählen und umgekehrt. So wird man bei Anwendung konzentrierter Säuren bei 0 bis 80°C, bevorzugt sogar bei 0 bis 60°C arbeiten. Umgekehrt kann bei Verwendung weniger hoch konzentrierter Säuren, beispielsweise solchen unter 80 Gew.-% (der Rest zu 100 % ist Wasser) im höheren Temperaturbereich arbeiten; so ist es beispielsweise möglich, in einem solchen Fall unter adiabatischen Bedingungen bis zu Temperaturen von 120 bis 150°C, bevorzugt 120 bis 140°C, zu arbeiten, um die Nitrierwärme zu nutzen (vgl. Houben-Weyl, 4. Auflage, Band 10/1, (1971), S. 479-488).

Die Ausbeute an Dinitroverbindung ist bei ausreichendem Angebot an Nitrierreagens praktisch quantitativ. Das Reaktionsprodukt wird in der Regel durch Abtrennen und Eindampfen der organischen Phase isoliert, Man kann allerdings auch in Lösungsmitteln arbeiten, in denen die Dinitroverbindung, insbesondere das 4,4′-Isomere, schwer löslich ist, beim Abkühlen ausfällt und leicht durch Filtration abgetrennt werden kann, Hierzu sind insbesondere die oben bereits genannten aliphatischen Kohlenwasserstoffe und die aliphatischen Halogenkohlenwasserstoffe geeignet.

Das rohe Nitrierprodukt kann unmittelbar zur nachfolgenden Reduktion eingesetzt werden. Man kann jedoch vor der Reduktion auch eine Auftrennung der Isomeren in der genannten Weise vornehmen und die Isomeren getrennt reduzieren.

Die Reduktion der Dinitro-diphenylpropane erfolgt nach an sich bekannten Methoden, Hierzu können Gemische von unedlen Metallen und Säuren, Gemische von Hydrazin und Hydrierkatalysatoren, Gemische von Sulfiden oder H₂S und Dithioniten oder katalytisch angeregter Wasserstoff eingesetzt werden. In bevorzugter Weise wird Hydrazin oder katalytisch angeregter Wasserstoff eingesetzt. Hydrierkatalysatoren sind beispielsweise Palladium oder Platin bzw. deren Oxide oder andere Edelmetalle auf Trägern, wie Kohle oder γ-Aluminiumoxid, oder Raney-Metalle, wie Raney-Nickel. Solche Hydrierkatalysatoren sind dem Fachmann beispielsweise aus Houben-Weyl, 4. Auflage, Band 11/1 (1957), S. 363-382, 394-406, 409-442, 454-457 und 462-472 bekannt.

Für den Fall der Hydrierung mit katalytisch angeregtem Wasserstoff kann diskontinuierlich oder kontonuierlich, beispielsweise im Batchverfahren oder in der Rieselphase, gearbeitet werden. Die Temperaturen betragen hierbei 0 bis 200°C, bevorzugt 20 bis 150°C. Die Wasserstoffdrücke betragen 1 bis 100 bar, bevorzugt 2 bis 50 bar, Es wird bei Wasserstoffüberschuß gearbeitet. Der Überschuß beträgt hierbei 0,2 bis 20 Mol H₂, bevorzugt 1 bis 10 Mol H₂ pro Mol Dinitro-diphenylpropan über die zur vollständigen Hydrierung der Nitrogruppen erforderliche Menge H₂ hinaus.

Das erhaltene Reduktionsprodukt wird vom Reduktionsmittel, Katalysatoren und den anderen Umsetzungsprodukten durch Filtrieren oder Auswaschen abgetrennt und kann für manche Zwecke, beispielsweise zur Phosgenierung zum Diisocyanat oder zur Epoxidharzhärtung, direkt verwendet werden, Bei besonderen Anforderungen an die Reinheit kann es in üblicher Weise durch Destillation oder Umkristallisieren gereinigt werden (vgl. US 3.670.094).

### Herstellung von Ausgangsprodukt

Zu einer Suspension von 35 g sauberem pulverisiertem Aluminiumchlorid in 3,5 l trockenem Benzol tropfte man bei 5°C innerhalb von 5 h 541 g 2-Chlor-2-phenylpropan (3,5 Mol) unter Rühren, hielt noch 15 Min. bei 5°C und goß dann auf Eis. Die Benzolphase wurde nach Abtrennen der wäßrigen Phase mit Pottasche neutralisiert, getrecknet und von überschüssigem Benzol befreit. Man erhielt 650 g Rohprodukt, das über eine ca. 100 cm hohe Kolonne fraktioniert destilliert wurde. Bei 152-160°C und 20 mbar gingen 527 g (78 % der theoretischen Ausbeute) 2,2-Diphenylpropan über.

### Beispiel, Stufe 1

Zu einer Lösung von 490 g (2,5 Mol) 2,2-Diphenylpropan in 500 ml Chloroform gab man 135 g Wasser und tropfte bei 0 bis 10°C innerhalb von 1-2 h unter Rühren eine Mischung von 935 g (6,75 Mol) handelsüblicher azeotrop siedender Salpetersäure und 2000 g konzentrierte Schwefelsäure zu, ließ noch 30 Min. nachreagieren, goß auf 3 kg Eis, trennte die Chloroformphase und extrahierte die wäßrige Phase noch zweimal mit Chloroform. Die vereinigten Chloroformphasen wurden mit Wasser gewaschen, mit Natriumsulfat getrocknet und eingedampft: 688 g Rohprodukt (99 % der theoretischen Ausbeute) mit einem N-Gehalt von 9,73 bis 9,80 (berechnet für Dinitroverbindung: 9,78 %) und einem Gehalt an 2,2-Bis-(4-nitrophenyl)-propan von 80 %. Durch Umkristallisieren aus Toluol, Xylol oder Cyclohexan/Tolüol konnte man das 4,4′-Isomere rein erhalten (Schmp. 135-6°C).

### Beispiel, Stufe 2

Zu 94 g (0,329 Mol) 2,2-Bis-(4-nitrophenyl)-propan in 250 ml Dioxan gab man 10 g Raney-Nickel, erwärmte auf 50°C und tropfte innerhalb von 1-2 h 84 g (1,3 Mol) 50 %iges Hydrazin hinzu. Nach Beendigung der exothermen Reaktion wurde auf 60°C erhitzt, bis die Gasentwicklung aufhorte. Das Gemisch wurde vom Raney-Nickel abfiltriert und eingedampft. Der Rückstand von 74 g (100 % der theoretischen Ausbeute) an braunem kristallinem Produkt wurde destillativ gereinigt. Bei 172-188°C/0,5 mbar gingen 72.g (97 % der theoretischen Menge) eines zu farblosen Kristallen erstarrenden Destillates über (Schmp. 129-131°C).

## Patentansprüche

1. Verfahren zur Herstellung von 2,2-Bis-(aminophenyl)-propan mit einem Anteil des 4,4′-Isomeren von mindestens 70 %, dadurch gekennzeichnet, daß man 2,2-Diphenylpropan in einem Lösungsmittel aus der Gruppe der aliphatischen Kohlenwasserstoffe und der aliphatischen Halogenkohlenwasserstoffe mit einem Nitriermittel aus der Reihe 50-100%ige HNO₃, HNO₃/H₂SO₄-Gemische (worin die HNO₃ bis zu 60 Gew.-% H₂O enthalten und H₂SO₄ bis zu 20 Gew.-% H₂O oder bis zu 20 Gew.-% SO₃ enthalten können) oder Gemische aus H₂SO₄ mit Nitraten der (Erd)alkalimetalle, wobei das molare Verhältnis von 2,2-Diphenylpropan zu dem die Nitrogruppe enthaltenden Nitrierreagens 1: (2-15) beträgt, zu 2,2-Bis-(nitrophenyl)-propan nitriert und dieses in einem nachfolgenden Schritt zu 2,2-Bis-(aminophenol)-propan reduziert.

2. Verfahren nach Anspruch 1, dadurch gekennzeichnet, daß das molare Verhältnis von 2,2-Diphenylpropan zu dem die Nitrogruppe enthaltenden Nitrierreagens 1:(2-5) beträgt.

3. Verfahren nach Anspruch 1, dadurch gekennzeichnet, daß die Nitrierung bei einer Temperatur von 0 bis 150°C durchgeführt wird.

4. Verfahren nach Anspruch 1, dadurch gekennzeichnet, daß zur Hydrierung Gemische von unedlen Metallen und Säuren, Gemische von Hydrazin und Hydrierkatalysatoren, Gemische von Sulfiden oder Schwefelwasserstoff und Dithioniten oder katalytisch angeregter Wasserstoff, bevorzugt Hydrazin oder katalytisch angeregter Wasserstoff, eingesetzt werden.

5. Verfahren nach Anspruch 4, dadurch gekennzeichnet, daß die Hydrierung mit katalytisch angeregtem Wasserstoff bei 0 bis 200°C, bevorzugt 20 bis 150°C und einem H₂-Druck von 1 bis 100 bar, bevorzugt 2 bis 50 bar, durchgeführt wird.

6. Verfahren nach Anspruch 5, dadurch gekennzeichnet, daß ein H₂-Überschuß von 0,2 bis 20 Mol H₂, bevorzugt 1 bis 10 Mol H₂ pro Mol Dinitro-diphenylpropan über die zur vollständigen Hydrierung der Nitrogruppen erforderliche Menge H₂ hinaus eingesetzt wird.

## Claims

1. Process for the preparation of 2,2-bis-(aminophenyl)-propane having a content of the 4,4′-isomer of at least 70%, characterized in that 2,2-diphenylpropane is nitrated in a solvent from the group comprising the aliphatic hydrocarbons and the aliphatic halogenohydrocarbons using a nitrating agent from the series comprising 50-100% strength HNO₃, HNO₃/H₂SO₄ mixtures (in which the HNO₃ can contain up to 60% by weight of H₂O and R₂SO₄ can contain up to 20% by weight of H₂O or up to 20% by weight of SO₃) or mixtures of H₂SO₄ with nitrates of the alkali metals or alkaline earth metals, the molar ratio of 2,2-diphenylpropane to the nitrating reagent containing the nitro group being 1:(2-15), to give 2,2-bis-(nitrophenyl)-propane and the latter is reduced in a subsequent step to 2,2-bis-(aminophenyl)-propane.

2. Process according to Claim 1, characterized in that the molar ratio of 2,2-diphenylpropane to the nitrating reagent containing the nitro group is 1:(2-5).

3. Process according to Claim 1, characterized in that the nitration is carried out at a temperature of 0 to 150°C.

4. Process according to Claim 1, characterized in that mixtures of base metals and acids, mixtures of hydrazine and hydrogenation catalysts, mixtures of sulphides or hydrogen sulphide and dithionites or catalytically activated hydrogen, preferably hydrazine or catalytically activated hydrogen, are employed for the hydrogenation.

5. Process according to Claim 4, characterized in that the hydrogenation is carried out using catalytically activated hydrogen at 0 to 200°C, preferably 20 to 150°C and at an H₂ pressure of 1 to 100 bar, preferably 2 to 50 bar.

6. Process according to Claim 5, characterized in that an H₂ excess of 0.2 to 20 mol of H₂, preferably 1 to 10 mol of H₂, per mole of dinitro-diphenylpropane over the amount of H₂ necessary for the complete hydrogenation of the nitro groups is employed.

## Revendications

1. Procédé de production de 2,2-bis(aminophényl)-propane avec une proportion de l'isomère 4,4′ d'au moins 70 %, caractérisé en ce qu'on effectue la nitration en 2,2-bis(nitrophényl)-propane du 2,2-diphénylpropane dans un solvant du groupe des hydrocarbures aliphatiques et des hydrocarbures aliphatiques halogénés avec un agent de nitration de la série de HNO₃ à 50-100 %, de mélanges HNO₃/H₂SO₄ (dans lesquels l'acide HNO₃ peut contenir jusqu'à 60 % en poids d'eau et l'acide H₂SO₄ peut contenir jusqu'à 20 % en poids d'eau ou jusqu'à 20 % en poids de SO₃) ou de mélanges de H₂SO₄ avec des nitrates de métaux alcalins ou alcalino-terreux, le rapport molaire du 2,2-diphénylpropane au réactif de nitration contenant le groupe nitro s'élevant à 1:(2-15) et on réduit le produit de nitration dans une étape subséquente en 2,2-bis(aminophényl)-propane.

2. Procédé suivant la revendication 1, caractérisé en ce que le rapport molaire du 2,2-diphénylpropane au réactif de nitration comprenant le groupe nitro a une valeur de 1:(2-5).

3. Procédé suivant la revendication 1, caractérisé en ce qu'on conduit la nitration à une température de 0 à 150°C.

4. Procédé suivant la revendication 1, caractérisé en ce qu'on utilise pour l'hydrogénation des mélanges de métaux non nobles et d'acides, des mélanges d'hydrazine et de catalyseurs d'hydrogénation, des mélanges de sulfures ou de sulfure d'hydrogène et de dithionites ou d'hydrogène catalytiquement activé, de préférence l'hydrazine ou de l'hydrogène catalytiquement activé.

5. Procédé suivant la revendication 4, caractérisé en ce qu'on conduit l'hydrogénation avec de l'hydrogène catalytiquement activé à 0-200°C, de préférence à 20-150°C et sous pression d'hydrogène moléculaire de 1 à 100 bars, de préférence de 2 à 50 bars.

6. Procédé suivant la revendication 5, caractérisé en ce qu'on utilise un excès d'hydrogène moléculaire de 0,2 à 20 moles de H₂, de préférence de 1 à 10 moles de H₂ par mole de dinitrodiphénylpropane en plus de la quantité de H₂ nécessaire pour l'hydrogénation totale des groupes nitro.
